Europäisches Patentamt

European Patent Office

Office européen des brevets

(11) Numéro de publication: **0 352 208**
**A1**

## (12) DEMANDE DE BREVET EUROPEEN

(21) Numéro de dépôt: **89440077.9**

(22) Date de dépôt: **17.07.89**

(51) Int. Cl.⁵: **A 61 F 13/15**

(30) Priorité: **18.07.88 FR 8810029**
**18.07.88 FR 8810030**

(43) Date de publication de la demande:
**24.01.90 Bulletin 90/04**

(84) Etats contractants désignés:
**AT BE CH DE ES FR GB GR IT LI LU NL SE**

(71) Demandeur: **CELATOSE Société anonyme dite:**
**47, Rue de Bradford**
**F-59200 Tourcoing (Nord) (FR)**

(72) Inventeur: **Bridge, Adrian Donald Clee**
**14 Monmouth Road**
**Abergavenny Gwent Wales (GB)**

(74) Mandataire: **Lepage, Jean-Pierre**
**Cabinet Lepage & Aubertin Innovations et Prestations**
**23/25, rue Nicolas Leblanc B.P. 1069**
**F-59011 Lille Cédex 1 (Nord) (FR)**

(54) **Couche-culotte.**

(57) L'invention est relative à une couche culotte qui trouvera notamment son application dans le domaine de l'hygiène infantile et/ou médicale.

La couche (1) est formée d'une feuille extérieure imperméable (5) d'une feuille intérieure perméable (6) et d'un matelas de matériau absorbant (7).

Elle présente en outre des moyens (15, 16) pour empêcher les remontées d'humidité, rendus élastiques, disposés au niveau de la ceinture (4), au moins de part et d'autre du matelas de matériau absorbant (7).

FIG.2

EP 0 352 208 A1

Description

L'invention est relative a une couche culotte qui trouvera notamment son application dans le domaine de l'hygiène infantile et/ou médicale.

Depuis de nombreuses années, on connait des couches culottes jetables. Leur aspect pratique et l'absence d'entretien ont largement contribué au développement de celles-ci qui ont fait l'objet de nombreux perfectionnements.

On connait de telles couches culottes formées d'une feuille extérieure imperméable, notamment étanche au liquide, généralement constituée par un film de polyéthylène, et d'une feuille intérieure perméable de confort, généralement en voile de non-tissé, entre lesquelles est disposé un matelas formé de matériau absorbant.

A ce sujet, des efforts particuliers ont été mis en oeuvre pour augmenter d'une part l'absorption de la couche culotte et d'autre part augmenter l'étanchéité. Le pouvoir d'absorption plus important permet notamment avec moins de matière de retenir une quantité de liquide importante. Toutefois, de ce fait, il est nécessaire que les couches présentent une certaine étanchéité pour éviter le rejet, vers l'extérieur de la couche, du liquide absorbé.

En ce qui concerne l'étanchéité, celle-ci a tout d'abord été renforcée au niveau de l'entrejambe du porteur et on a prévu différents systèmes élastiques d'entrejambes qui permettent d'adapter la forme de la couche à la morphologie du porteur.

Par ailleurs, au niveau de la ceinture de la dite couche culotte, une première amélioration est apparue avec la présence de dispositifs de fermeture adhésifs, permettant le décollage et le recollage de ceux-ci, sans détériorer la feuille extérieure, afin de pouvoir ajuster au mieux les dimensions de la ceinture aux mensurations du porteur.

D'autres fabricants ont proposé, en complément de ces systèmes adhésifs, une ceinture élastique qui permet de rendre la taille extensible devant ou derrière et autorise les variations dans le temps de la morphologie du porteur. En effet, dans le cas d'un bébé par exemple, les variations de la taille entre deux repas sont très sensibles; la ceinture élastique permet alors de pallier ces variations sans être obligé de relanger le bébé.

A ce sujet, on connait notamment une première réalisation dans laquelle est placée une bande de mousse élastique, telle qu'une mousse de polyuréthane, apte à se rétracter naturellement pour former la dite ceinture élastique.

On connait également une autre réalisation qui prévoit au niveau de la partie avant et/ou la partie arrière de la couche un ruban thermo-rétractile, sensible à la chaleur pour provoquer sa rétraction et procurer l'élasticité requise.

Particulièrement ce ruban est formé de deux films de polymère prenant en sandwich plusieurs brins élastiques solidarisés notamment par soudures sur les deux dits films. L'effet de la chaleur sur le ruban permettra de désolidariser les films et les brins élastiques rendant à ceux-ci leur effet naturel.

Ces différentes réalisations de couches culottes avec ceinture élastique ont donc pour but d'éviter certaines fuites en adaptant la ceinture de la couche à la taille du porteur afin d'augmenter le contact et d'éviter les entrebaillements néfastes.

En effet, de telles couches à ceinture élastique permettent lorsque la quantité de liquide répandue sur le tampon instantanément est importante, une bonne application de la matière absorbante sur la peau du porteur et évite les écoulements intempestifs hors de la couche pour pallier l'inertie d'absorption du tampon absorbant.

Toutefois, ces dispositifs ne permettent pas d'éviter les remontées d'humidité au niveau de la taille rendues possibles de par le grand pouvoir de rétention de liquide des matières utilisées en tant que tampon absorbant.

Pour pallier les remontées d'humidité à la taille, d'autres techniques ont été développées et on connait notamment une couche culotte qui est équipée au niveau de la ceinture d'une feuille étanche prévue en extrémité du matelas de matériau absorbant pour diminuer les réjections de liquide.

A cet égard, un morceau de film de polymère imperméable est placé entre le voile de non-tissé de la feuille intérieure perméable et la feuille extérieure imperméable en recouvrant une partie du matelas absorbant du côté du porteur. Ce film est maintenu en place par divers moyens de fixation, tels que collage, liage par embossage, soudure par ultrason, liant le dit film de polymère imperméable et le voile de non-tissé.

Toutefois, dans de telles réalisations, certains inconvénients subsistent au niveau de l'étanchéité et ne donnent pas une parfaite efficacité aux moyens utilisés.

En effet, d'une part, le film de polymère n'est pas fixé d'une manière totalement continue sur les matériaux, ce qui provoque des passages préférentiels pour les écoulements notamment vers le haut, et d'autre part, ces remontées d'humidité sont bloquées uniquement lorsque le porteur est en position couchée, c'est-à-dire lorsque la ceinture est positionnée bien à plat sur la taille de celui-ci.

Le but de la présente invention est de proposer une couche culotte, qui trouvera notamment son application dans le domaine de l'hygiène infantile et/ou médicale, qui permette de pallier les inconvénients précités et qui autorise une augmentation du confort du porteur en évitant les remontées d'humidité au niveau de la taille en empêchant que le liquide ne sorte au niveau de la ceinture vers le haut et/ou vers l'intérieur de la couche.

Un des buts de la présente invention est de proposer une couche culotte dont la structure permet toutefois sa fabrication selon des procédés en continu.

Un autre but de la présente invention est de proposer une couche culotte équipée de moyens spécifiques qui évitent les remontées d'humidité quelle que soit la morphologie instantanée du porteur et quelle que soit sa position debout ou couchée.

D'autres buts et avantages de la présente invention apparaitront au cours de la description qui va suivre qui n'est cependant donnée qu'à titre indicatif et qui n'a pas pour but de la limiter.

Selon la présente invention, la couche culotte, qui trouvera notamment son application dans le domaine de l'hygiène infantile et/ou médicale, formée d'une partie avant, et d'une partie arrière, réunies par une partie centrale constituant l'entrejambe, la dite couche présentant :
- une feuille extérieure imperméable, étanche au moins au liquide,
- une feuille intérieure perméable de confort,
- un matelas de matériau absorbant, disposé entre les deux dites feuilles,
- des moyens de fermeture, prévus au niveau des dites parties avant et/ou arrière, afin de constituer une ceinture pour la dite couche,
est caractérisée par le fait qu'elle comporte des moyens pour empêcher les remontées d'humidité, rendus élastiques, disposés au niveau de la ceinture au moins de part et d'autre du matelas de matériau absorbant, évitant que le liquide absorbé ne ressorte au niveau de la ceinture vers le haut et/ou vers l'intérieur de la couche.

L'invention sera mieux comprise à la lecture de la description suivante, accompagnée des figures qui en font partie intégrante.

Les figures 1a et 1b montrent schématiquement une couche culotte réalisée selon la présente invention et illustrent respectivement la face intérieure et la face extérieure de la couche.

La figure 2 représente une vue en perspective d'une couche culotte selon la présente invention en position d'utilisation.

La figure 3 montre une vue en coupe partielle de la couche représentée à la figure 1a, selon l'axe III-III.

La figure 4 représente une vue partielle schématique agrandie de la partie arrière de la couche culotte de la figure 1, vue côté intérieure.

La présente invention vise donc une couche culotte qui trouvera notamment son application dans le domaine de l'hygiène infantile et/ou médicale.

On a rappelé que de telles couches culottes ont subi un développement très important grâce à leur facilité d'utilisation et leur commodité d'emploi.

Toutefois, les perfectionnements en matière de qualité de matériau absorbant ont développé la recherche dans d'autres domaines notamment, pour augmenter l'étanchéité des couches culottes au niveau de l'entrejambe et de la ceinture.

En effet, au niveau de l'étanchéité, deux problèmes différents se posent. Le premier apparait lors de l'absorption instantanée du matériau absorbant et il est nécessaire d'éviter que le liquide ne s'écoule à l'extérieur de la couche, celui-ci ne pouvant pas être absorbé instantanément du fait de l'inertie du matériau absorbant.

Le deuxième problème réside au niveau des remontées d'humidité aux entrejambes et à la ceinture, remontées d'humidité provoquées par exemple par des mouvements du porteur lorsque le matériau absorbant a quasiment atteint son degré d'absorption maximum.

De plus, étant donnée la structure de la couche, on facilite l'absorption longitudinale du matériau absorbant pour autoriser un cheminement du liquide déposé au niveau de l'entrejambe de la couche vers les parties avant et/ou arrière. De ce fait, lorsque la quantité de liquide est importante, toute pression sur le matériau absorbant provoque une remontée de liquide qui aura tendance à s'échapper au niveau de la ceinture par l'intérieur et par le haut de la couche culotte.

La présente invention permet de résoudre ces deux impératifs, et à ce sujet, comporte des moyens pour empêcher les remontées d'humidité au niveau de la ceinture vers le haut et/ou vers l'intérieur.

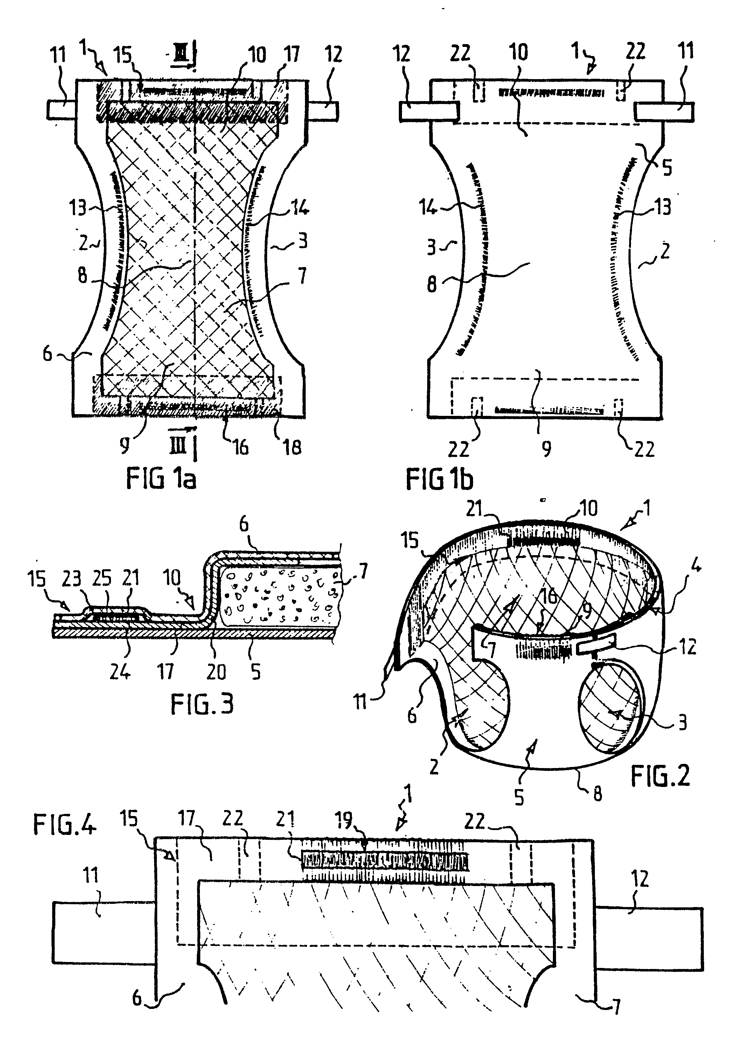

Les figures 1 et 2 illustrent une couche culotte 1 de type traditionnel qui comporte des découpes latérales 2 et 3 pour permettre le passage des jambes et qui peut être pliée telle qu'elle forme une ceinture 4 apte à s'adapter à la morphologie du porteur.

Pour ce qui est de la structure proprement dite de la couche culotte 1, elle est formée traditionnellement par une feuille extérieure 5, imperméable, étanche au moins au liquide, généralement réalisée par un film de polyéthylène, ainsi qu'une feuille intérieure perméable de confort 6, destinée à s'appliquer vers l'utilisateur, réalisée généralement en voile de non-tissé.

Entre la feuille extérieure imperméable 5 et la feuille intérieure perméable 6, est disposé un matelas central de matériau absorbant 7, généralement constitué d'ouate de cellulose ou tout autre matériau absorbant voire même hyperabsorbant. A cet égard, pour former une enveloppe dans laquelle est renfermé le dit matériau absorbant 7, les feuilles extérieure 5 et intérieure 6 sont fixées mutuellement sur la périphérie de la couche culotte 1.

Ainsi formée, la couche culotte 1 montre une partie centrale 8, présentant les dites découpes 2 et 3 pour constituer l'entrejambe, réunissant d'une part une partie avant 9 et d'autre part une partie arrière 10.

En outre, pour constituer la ceinture 4, la couche culotte 1 présente des moyens de fermeture 11, 12, notamment prévus au niveau des parties avant 9 et/ou arrière 10. Dans le cas représenté sur les figures, ces moyens de fermeture sont avantageusement constitués par des dispositifs adhésifs fixés sur la partie arrière de la couche 10. De tels moyens sont connus de l'Homme de l'Art.

Par ailleurs, pour l'étanchéité, au niveau de l'entrejambe, la couche 1 comporte des élastiques longitudinaux 13, 14 disposés de part et d'autre du dit matelas de matériau absorbant 7 au niveau de la partie centrale 8.

Pour ce qui est de l'étanchéité au niveau de la ceinture, la couche culotte 1 de la présente invention comporte des moyens 15, 16 pour empêcher les remontées d'humidité, rendus élastiques, disposés au niveau de la ceinture 4 au moins de part et d'autre du matelas de matériau absorbant 7. Ces moyens évitent que le liquide, déjà absorbé par la couche, ne ressorte au niveau de la ceinture vers le haut et/ou vers l'intérieur de la couche lors des mouvements du porteur.

Plus précisément, selon un mode de réalisation, les dits moyens 15, 16 pour empêcher les remontées d'humidité sont constitués par une barrière 17 ou 18 étanche, au moins au liquide, disposée transversalement au niveau de la partie avant 9 et de la partie arrière 10, et rendue élastique sur au moins une portion 19 de la partie avant 9 et/ou arrière 10. Une telle réalisation est particulièrement illustrée aux figures 3 et 4.

Ainsi, selon la présente invention, on réalise non seulement une barrière étanche qui permettra d'éviter les remontées d'humidité lorsque la couche sera placée dans de bonnes conditions d'utilisation, mais de plus, cette barrière étanche, étant rendue élastique, augmentera l'efficacité des moyens 15, 16.

En effet, la fiabilité de tels moyens dépend d'une part de la bonne étanchéité réalisée au niveau de la ceinture et d'autre part de l'application de celle-ci au niveau de la taille du porteur.

Par ailleurs, il est à remarquer que le fait de placer une barrière d'étanchéité transversalement au niveau de la ceinture va diminuer la souplesse de la couche à ce niveau; toutefois, grâce à l'élastification, on va redonner à la ceinture une certaine souplesse qui est nécessaire pour la bonne efficacité des moyens pour empêcher les remontées d'humidité.

Dans un premier mode de réalisation de la présente invention, la dite barrière étanche élastique 17 ou 18 se présente sous la forme d'un film 20 de matière rapportée, disposé transversalement et partiellement au niveau de la partie avant 9 et arrière 10 ainsi que de moyens d'élastification 21 placés à l'état tendu sur au moins une portion 19 du dit film rapporté 20, ce au niveau de la partie avant 8 et/ou de la partie arrière 9.

En effet, il est à remarquer que, pour rendre élastiques les moyens 15, 16, pour empêcher les remontées d'humidité, il peut être suffisant de placer les moyens d'élastification 21 seulement sur une partie de la périphérie de la ceinture, soit à l'avant, soit à l'arrière de la couche. Néanmoins, pour plus d'efficacité, on placera ces moyens d'élastification au niveau de la partie avant et arrière.

Le dit film 20 peut être notamment constitué par un film de colle étanche tel qu'une enduction de polymère adhésif sur la feuille intérieure 6 de non-tissé avant qu'il ne soit associé au matelas de matériau absorbant 7 au cours du processus de fabrication.

A titre d'exemple, cet adhésif polymère peut être une colle "hot melt" sensible à la pression, filmogène et à haute cohésion.

En ce qui concerne le processus de fabrication, le dépôt de la colle sera synchronisé avec le défilement de la bande complexe destinée à former les couches, afin d'appliquer sur les extrémités des couches la dite colle avec un recouvrement du tampon absorbant et de la feuille imperméable extérieure.

Grâce à l'utilisation de cette technique, les dépôts de colle se lient parfaitement à la feuille extérieure imperméable 5 et procurent un scellage étanche des extrémités.

Cela étant, lorsque la dite couche culotte 1 est équipée d'élastiques longitudinaux 13, 14, au niveau de l'entrejambe 2, 3, l'enduction de colle sera telle que l'on prévoit des réservations pour permettre la technique de retrait des élastiques.

En effet, généralement, les couches culottes 1 sont obtenues à partir d'une bande complexe, fabriquée en continu à partir des éléments de base, à savoir film imperméable 5 pour former la feuille extérieure 5, voile de non-tissé pour former la feuille intérieure 6, et matelas de matériau absorbant 7.

Les élastiques longitudinaux 13, 14 sont placés de part et d'autre du matelas 7, à l'état tendu, et sont collés sur une certaine portion pour former l'entrejambe.

Entre ces portions, les élastiques ne sont pas collés mais restent tendus sur la bande complexe, pendant tout son trajet. A l'issue du cycle de fabrication, les couches culottes sont obtenues par sectionnement de la bande, ce qui provoque automatiquement le sectionnement des élastiques. A ce moment, il est donc nécessaire que les parties d'élastique non collées puissent se rétracter afin d'obtenir les fronces souhaitées seulement au niveau de l'entrejambe.

C'est pourquoi, selon la présente invention, les dits moyens pour empêcher les remontées d'humidité présentent des zones de passage 22 des dits élastiques longitudinaux 13, 14 pour autoriser leur rétractation après la découpe de la dite bande en continu et par suite après la découpe des dits élastiques.

Pour constituer ces zones de passage 22, on enduira, par exemple, les élastiques 13, 14 d'une substance anti-collante dans ces zones pour éviter que les élastiques ne collent au niveau de la barrière étanche de colle 20.

Par ailleurs, dans un autre mode de réalisation, on pourrait envisager de remplacer le film de colle 20 formant la dite barrière étanche 17 ou 18 par un film de polymère, au moins étanche au liquide, disposé transversalement au niveau de la partie avant 9 et arrière 10 de façon similaire.

Ce film de polymère devra néanmoins être maintenu en place par des moyens de solidarisation adaptés tels que collage, liage par embossage, soudure par ultrason, selon des techniques connues.

De plus, comme décrit ci-dessus, le film de polymère sera rendu élastique, en plaçant et en fixant, à l'état tendu, sur au moins une portion du dit film de polymère étanche, des moyens d'élastification, ce au niveau de la partie avant et/ou arrière de la couche.

En outre, dans les différents cas de réalisation, on placera avantageusement la barrière 17 ou 18 telle qu'elle soit disposée en chevauchant l'extrémité du matelas 7 et la feuille perméable 5, la longueur de la dite barrière 20 étant au moins égale à la largeur de la partie avant 9 et/ou arrière 10 du matelas de matériau absorbant 7.

En ce qui concerne la réalisation proprement dite des moyens d'élastification 21, différentes techniques peuvent être utilisées.

Par exemple, la portion 19 des dits moyens 21 pourrait être constituée par une bande de mousse élastique ou un morceau de polyéthylène élastique. Ces deux éléments devraient être préalablement tendus, pour être posés à l'état étiré, sur la couche, et autorisés par rétractation le fronçage de la barrière étanche.

Dans un autre mode de réalisation, tel qu'illustré à la figure 3, les moyens d'élastification 21 se présenteront sous la forme d'un ruban complexe thermo-rétractile qui est préalablement à l'état tendu et qui peut être activé ensuite par un apport d'énergie pour provoquer la rétractation.

A ce sujet, on connait un tel type de ruban thermo-rétractile constitué par des brins 25 de polyuréthane pré-étirés entre deux films de polymère 23, 24, ces brins 25 étant fixés notamment par soudure aux deux dits films 23, 24. La fixation d'un tel ruban thermo-rétractile se fera notamment par collage ou soudure.

Après sa pose, sous l'action de la chaleur, on désoude les brins élastiques des films de polymère et on obtient ainsi la rétractation des élastiques.

De plus, comme le montrent les figures, les dits moyens d'élastification 21 sont placés avantageusement de part et d'autre du matelas absorbant 7; on obtient alors un fronçage efficace de la barrière d'étanchéité 17, 18 au niveau du sandwich : feuille imperméable 5, film d'étanchéité 20, feuille perméable 6.

Ces solutions sont toutefois non limitatives et on pourrait envisager d'utiliser tout type d'élastiques naturels ou synthétiques.

Dans le mode de réalisation précédent, la barrière étanche est constituée de deux éléments structurellement différents, à savoir par exemple un film de colle étanche et des moyens d'élastification substantiellement constitués par une mousse élastique, du polyéthylène élastique ou un ruban complexe thermo-rétractile.

Toutefois, il pourrait être envisagé dans un autre mode de réalisation de réaliser la dite barrière étanche élastique 17, 18 par un seul et même matériau réalisant alors les deux fonctions étanchéité et élasticité.

Dans ce cas, la dite barrière étanche élastique se présente sous la forme d'une bande de colle étanche élastique, disposée transversalement et partiellement au niveau de la partie avant 9 et arrière 10, comme décrit précédemment, c'est-à-dire entre la dite feuille perméable intérieure 6 et d'une part le dit matelas de matériau absorbant 7 et/ou d'autre part la dite feuille externe imperméable 5.

Plus précisemment, la dite bande de colle étanche élastique se présente sous la forme d'un matériau polymère élastomérique et auto-adhésif du type "hot melt".

Après refroidissement, cette matière adhésive est élastique lorsqu'elle est sous tension.

En outre, elle permet après sa pose, et après un calandrage de réaliser le collage de la feuille perméable intérieure 6 et de la feuille externe imperméable 5 en constituant entre celles-ci une barrière étanche élastique.

Cela étant, cette bande est obtenue par extrusion ou limite d'extrusion puis soit placée à l'état tendu sur un support non plissé, tel que par exemple sur la feuille imperméable non plissée, soit dans un état non tendu sur un support plissé au moins partiellement, tel que par exemple sur la feuille externe imperméable au moins partiellement plissée.

En conclusion, grâce à la structure de la couche de la présente invention, au niveau de la ceinture, on dispose ainsi d'une barrière étanche élastique dont l'efficacité est renforcée, ce afin d'éviter les remontées d'humidité par le haut et/ou par l'intérieur de la couche.

En effet, les moyens d'élastification de la barrière permettent d'appliquer celle-ci sur la taille du porteur, ce quelle que soit sa morphologie et les variations de celle-ci pendant l'utilisation de la couche.

En effet, il se peut qu'au moment de la pose de la couche, la taille soit plus importante et qu'au cours du temps, cette dimension diminue sensiblement. Dans ce cas, la barrière d'humidité pourra rester efficace car elle sera toujours adaptée aux dimensions de la taille.

De même, si les variations de la taille de l'individu sont dus à son positionnement couché, assis ou debout, on aura également une adaptation parfaite au niveau de la ceinture de la couche pour renforcer l'efficacité des moyens empêchant les remontées d'humidité.

Naturellement, d'autres mises en oeuvre de la présente invention, à la portée de l'Homme de l'Art, auraient pu être envisagées sans pour autant sortir du cadre de la présente invention.

**Revendications**

1. Couche culotte (1) qui trouvera notamment son application dans le domaine de l'hygiène infantile et/ou médicale, formée d'une partie avant (9), et d'une partie arrière (10), réunies par une partie centrale (8) constituant l'entrejambe (3), la dite couche présentant :
- une feuille extérieure imperméable (5) étanche au moins au liquide,
- une feuille intérieure perméable (6) de confort,
- un matelas de matériau absorbant (7), disposé entre les deux dites feuilles (5, 6),
- des moyens de fermeture (11, 12), prévus au niveau des parties avant et/ou arrière, afin de constituer une ceinture (4) pour la dite couche (1),
caractérisée par le fait qu'elle comporte des moyens (15, 16) pour empêcher les remontées d'humidité, rendus élastiques, disposés au niveau de la ceinture (4), au moins de part et d'autre du matelas absorbant (7), évitant que le liquide absorbé ne ressorte au niveau de la ceinture vers le haut et/ou vers l'intérieur de la couche.

2. Couche culotte selon la revendication 1 caractérisée par le fait que les dits moyens (15, 16) pour empêcher les remontées d'humidité sont constituées par une barrière étanche (17 ou 18), au moins au liquide, disposée transversalement au niveau de la partie avant (9) et de la partie arrière (10), et rendue élastique sur au moins une portion (19) de la partie avant (9) et/ou arrière (10).

3. Couche culotte selon la revendication 2, caractérisée par le fait que la dite barrière étanche élastique (17 ou 18) se présente sous la forme :

- d'un film (20) de colle étanche, disposé transversalement et partiellement au niveau de la partie avant (9) et arrière (10), entre la dite feuille perméable intérieure (6) et, d'une part le dit matelas de matériau absorbant (7), et/ou d'autre part la dite feuille externe imperméable (5),

- de moyens d'élastification (21), placés à l'état tendu, sur au moins une portion (19) du dit film (20), au niveau de la partie avant (9) et/ou de la partie arrière (10).

4. Couche culotte selon la revendication 2, caractérisée par le fait que la dite barrière étanche élastique (17 ou 18) se présente sous la forme :

- d'un film de polymère, au moins étanche au liquide, disposé transversalement au niveau de la partie avant (9) et arrière (10), entre la dite feuille perméable intérieure (6) et, d'une part, le dit matelas de matériau absorbant (7), et/ou, d'autre part, la dite feuille externe imperméable (5) maintenu en place par des moyens de solidarisation adaptés,

- de moyens d'élastification (21), placés et fixés à l'état tendu, sur au moins une portion du dit film de polymère étanche, au niveau de la partie avant (9) et/ou de la partie arrière (10).

5. Couche culotte selon la revendication 2, caractérisée par le fait que la dite barrière étanche élastique (17 ou 18) se présente sous la forme d'une bande de colle étanche élastique, disposée transversalement et partiellement au niveau de la partie avant (9) et arrière (10), entre la dite feuille perméable intérieure (6) et d'une part le dit matelas de matériau absorbant (7), et/ou d'autre part la dite feuille externe imperméable (5).

6. Couche culotte selon la revendication 2, caractérisée par le fait que les dits moyens d'élastification (21) se présentent sous la forme d'une mousse élastique, ou sous la forme de polyéthylène élastique, ou sous la forme d'un ruban complexe thermo-rétractile, constitué de brins élastiques (25) étirés et fixés entre deux films de polymère (23, 24).

7. Couche culotte selon la revendication 1, la dite couche (1) étant obtenue à partir d'une bande en continu complexe, et présentant en outre des élastiques longitudinaux (13, 14) disposés de part et d'autre du dit matelas de matériau absorbant (7), au niveau de l'entrejambe (2, 3), caractérisée par le fait que les dits moyens (15, 16) pour empêcher les remontées d'humidité présentent des zones de passage (22) des dits élastiques longitudinaux (13, 14) pour autoriser leur rétractation après la découpe de la dite bande en continu et par suite de la découpe des dits élastiques.

8. Couche culotte selon la revendication 3 ou 4, caractérisée par le fait que la longueur de la dite barrière est au moins égale à la largeur de la partie avant et/ou arrière du matelas de matériau absorbant, et que la barrière est disposée telle qu'elle chevauche l'extrémité du matelas et de la feuille imperméable.

9. Couche culotte selon la revendication 3, caractérisée par le fait que le dit film de colle (20) se présente sous la forme d'un adhésif "hot melt" sensible à la pression, filmogène et à haute cohésion

10. Couche culotte selon la revendication 5, caractérisée par le fait que la dite bande de colle étanche élastique se présente sous la forme d'un matériau, polymère élastomérique et auto-adhésif du type "hot melt".

FIG 1a

FIG 1b

FIG. 3

FIG. 2

FIG. 4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-252413 (KAO CORPORATION)<br>* page 4, dernier alinéa - page 6, alinéa 3; revendications 1-3; figures 1-4 * | 1 | A61F13/15 |
| A | | 2, 4, 8 | |
| | ---- | | |
| X | EP-A-197736 (THE PROCTER & GAMBLE COMPANY)<br>* page 11, ligne 6 - page 12, ligne 23; revendications 1-5; figures 1-4 * | 1 | |
| A | | 2, 4, 7, 8 | |
| | --- | | |
| Y | EP-A-242766 (PAUL HARTMANN AG)<br>* abrégé; figure 1 * | 1 | |
| A | | 2-10 | |
| | --- | | |
| Y | FR-A-2462112 (CENTRE D'ETUDES TECHNIQUES ET DE REALISATIONS APPLIQUEES S.A.)<br>* page 8, ligne 17 - page 9, ligne 9; figure 1 * | 1 | |
| A | | 2-4, 6 | |
| | --- | | |
| A | FR-A-2425205 (CONSORTIUM GENERAL TEXTILE)<br>* figure 1 * | 7 | DOMAINES TECHNIQUES RECHERCHES (Int. Cl.5) |
| | --- | | |
| A | US-A-4430086 (JOHNSON & JOHNSON BABY PRODUCTS) | | A41B |
| | ------ | | |

Le présent rapport a été établi pour toutes les revendications

| Lieu de la recherche | Date d'achèvement de la recherche | Examinateur |
|---|---|---|
| LA HAYE | 17 OCTOBRE 1989 | KARIPIDOU C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
...............................................................
& : membre de la même famille, document correspondant

EPO FORM 1503 03.82 (P0402)